# EUROPEAN PATENT APPLICATION

(11) **EP 2 853 232 A1**
(43) Date of publication of application: **01.04.2015**
(21) Application number: 14193752.4
(22) Date of filing: 10.08.2011
(51) Int. Cl.: A61F 2/00, A61B 17/00

(54) **Barbed implantable devices**

(30) Priority: 10.08.2010 US 372329 P
(62) Divisional of application: 11816973.9
(71) Applicant: Covidien LP, Mansfield, MA 02048 (US)
(72) Inventor: Thomas, Jonathan, New Haven, CT Connecticut 06511 (US); Sargeant, Timothy, Guilford, CT Connecticut 06437 (US)
(74) Representative: Soames, Candida Jane

(57) **Abstract**

An implantable medical device comprising a biocompatible substrate having a surface comprising at least one barbed and spiked nap (302), wherein the nap has a free end, characterised in that: the spike is formed on the free end of the nap.

## Description

### BACKGROUND

### Technical Field

The present disclosure relates generally to implantable medical devices having at least one tissue gripping element and to methods for forming such devices.

### Background of Related Art

Surgical meshes may be used during both laparoscopic and open surgery for repair of many types of defects and injuries. For example, surgical meshes are commonly used in the repair of hernias. The meshes may be used to provide support to surrounding tissue, as well as to supplement standard suturing.

During hernia repair, the mesh may be placed over the entirety of damaged tissue and some of the healthy tissue surrounding the defect. The mesh can be held in place by a fixation device that attaches the mesh to the surrounding tissue. A variety of different fixation devices may be used to anchor the mesh into the tissue. For example, a needled suture may be passed through or around the tissue near the defect to hold the mesh in a position which spans the injured tissue. In other instances, staples, tacks, clips and pins are known to be passed through or around the tissue near the defect to anchor the implant in a position which spans the injured tissue.

Unfortunately, the use of such fixation devices may increase the patient's discomfort and, in certain instances, may weaken the tissue to which the fixation devices are attached. Certain techniques involve placing a mesh against the repair site without the addition of a fixation device. For example, in some instances the mesh may be simply positioned within the abdomen allowing the pressure of the peritoneum to hold the mesh against the posterior side of the abdominal wall. However, fixation of the mesh may be helpful in order to avoid folding, shrinkage, and migration of the mesh.

Although methods that require the use of fixation devices have been proven effective in anchoring an implant such as a mesh into the tissue, penetration of the tissue by such devices inflicts additional trauma to the damaged tissue or the tissue near the defect and requires additional time for healing. Thus, implantable devices which do not require the use of sutures, staples, tacks, pins, and/or clips is desirable in order to further limit the amount of trauma to healthy tissue surrounding the wound and caused by the fixation devices.

### SUMMARY

Accordingly, the present disclosure describes implantable medical devices which include at least one tissue-gripping element, such as a barbed loop or a barbed and spiked nap.

In certain embodiments, the implantable medical devices include a biocompatible substrate having a surface containing at least one barbed loop. The at least one barbed loop may protrude perpendicularly from the surface of the biocompatible substrate. In embodiments, a plurality of barbed loops may be positioned along any portion of the surface of the biocompatible substrate.

In some embodiments, the implantable medical devices described herein include a biocompatible substrate having a surface containing barbed and spiked naps. The barbed and spiked naps may protrude perpendicularly from the surface of the biocompatible substrate. In embodiments, the barbed and spiked naps may be positioned along any portion of the surface of the biocompatible substrate.

Methods of forming such devices are also disclosed. For instance, in certain embodiments, methods of forming a barbed implantable medical device are described which include: providing at least one barbed, biocompatible filament; and, combining the at least one barbed biocompatible filament with a biocompatible substrate to form barbed loops along a surface of the biocompatible substrate. Such methods produce barbed implantable medical devices which include tissue-gripping elements such as barbed loops.

In other embodiments, methods of forming a barbed implantable medical device are described which include: providing a biocompatible substrate having loops protruding perpendicularly from a surface of the biocompatible substrate; and forming barbs on the loops of the medical device.

In addition, methods of forming an implantable medical device having barbed and spiked naps are also described which include: providing a biocompatible substrate having loops protruding perpendicularly from a surface of the biocompatible substrate; forming barbs on the loops of the medical device; and treating a portion of the loops to melt and separate each loop into two barbed and spiked naps.

In some embodiments, methods of forming an implantable medical device having barbed and spiked naps are also described which include: providing at least one barbed, biocompatible filament; combining the at least one barbed biocompatible filament with a biocompatible substrate to form barbed loops along a surface of the of the biocompatible substrate; and, treating a portion of the barbed loops to melt and separate each barbed loop into two barbed and spiked naps.

### BRIEF DESCRIPTION OF THE DRAWINGS

The accompanying drawings, which are incorporated in and constitute a part of the specification, illustrate embodiments of the disclosure and, together with a general description of the disclosure given above, and the detailed description of the embodiments given below, serve to explain the principles of the disclosure.
FIG. 1 is a side view of an implantable medical device having a biocompatible substrate containing barbed loops according to one embodiment described in the present disclosure;
FIG. 2 is a side view of an implantable medical device having a biocompatible substrate containing barbed loops according to another embodiment described in the present disclosure;
FIG. 3 is a side view of an implantable medical device having a biocompatible substrate containing barbed and spiked naps according to yet another embodiment described in the present disclosure;
FIG. 4 is a side view of an implantable medical device having a biocompatible substrate containing barbed and spiked naps according to still another embodiment described in the present disclosure;
FIG. 5 is a side view of an implantable medical device having a biocompatible substrate containing barbed loops and barbed and spiked naps according to still another embodiment described in the present disclosure;
FIG. 6 is a top view of an implantable medical device having a biocompatible substrate containing tissue-gripping elements according to still another embodiment described in the present disclosure;
FIG. 7 is a top view of an implantable medical device having a biocompatible substrate containing tissue-gripping elements according to still another embodiment described in the present disclosure;
FIG. 8 is a diagram showing a weave pattern for forming an implantable medical device according to an embodiment described in the present disclosure; and,
FIG. 9 is a diagrammatic side view of a device permitting the formation of barbed loops and/or barbed and spiked naps on a medical device.

### DETAILED DESCRIPTION

The present disclosure relates to barbed implantable medical devices which display tissue-gripping capabilities. In certain embodiments, the implantable medical devices include at least one barbed loop to attach to tissue. In other embodiments, the implantable medical devices include at least one barbed and spiked nap to attach to tissue. In still other embodiments, the implantable medical devices include at least one barbed loop and at least one barbed and spiked nap to attach to tissue.

The implantable medical devices include a biocompatible substrate having a surface to which the barbed loops, barbed and spiked naps, or a combination of the two may be positioned. The biocompatible substrates are often planar in configuration, however, any two-dimensional or three dimensional shapes suitable for implantation may be used. Some examples of suitable biocompatible substrates include films, foams, meshes, buttresses, patches, tapes, pledgets, occlusion devices, and the like. In certain embodiments, the biocompatible substrate is a surgical mesh.

Any biocompatible material may be used to form the biocompatible substrates and/or the filaments described herein. For example, the substrate may be made from natural, synthetic, bioabsorbable or non-bioabsorbable materials. It should of course be understood that any combination of natural, synthetic, bioabsorbable and non-bioabsorbable materials may be used to form the substrates or filaments described herein. The term "bioabsorbable" as used herein is defined to include both biodegradable and bioresorbable materials. By bioabsorbable, it is meant that the materials decompose, or lose structural integrity under body conditions (e.g. enzymatic degradation or hydrolysis) or are broken down (physically or chemically) under physiologic conditions in the body such that the degradation products are excretable or absorbable by the body.

Representative natural bioabsorbable materials include: polysaccharides, such as alginate, dextran, chitin, hyaluronic acid, cellulose, collagen, gelatin, fucans, glycosaminoglycans, and chemical derivatives thereof (substitutions and/or additions of chemical groups, for example, alkyl, alkylene, hydroxylations, oxidations, and other modifications routinely made by those skilled in the art); and proteins, such as albumin, casein, zein, silk, and copolymers and blends thereof, alone or in combination with synthetic polymers.

Synthetically modified natural polymers include cellulose derivatives, such as alkyl celluloses, hydroxyalkyl celluloses, cellulose ethers, cellulose esters, nitrocelluloses, and chitosan. Examples of suitable cellulose derivatives include methyl cellulose, ethyl cellulose, hydroxypropyl cellulose, hydroxypropyl methyl cellulose, hydroxybutyl methyl cellulose, cellulose acetate, cellulose propionate, cellulose acetate butyrate, cellulose acetate phthalate, carboxymethyl cellulose, cellulose triacetate, and cellulose sulfate sodium salt. These are collectively referred to herein as "celluloses."

Representative synthetic bioabsorbable polymers include polyhydroxy acids prepared from lactone monomers, such as glycolide, lactide, caprolactone, ε-caprolactone, valerolactone, and δ-valerolactone, as well as pluronics, carbonates (e.g., trimethylene carbonate, tetramethylene carbonate, and the like), dioxanones (e.g., 1,4-dioxanone and p-dioxanone), 1,dioxepanones (e.g., 1,4-dioxepan-2-one and 1,5-dioxepan-2-one), and combinations thereof. Polymers formed therefrom include: polylactides; poly(lactic acid); polyglycolides; poly(glycolic acid); poly(trimethylene carbonate); poly(dioxanone); poly(hydroxybutyric acid); poly(hydroxyvaleric acid); poly(lactide-co-(ε-caprolactone-)); poly(glycolide-co-ε-caprolactone)); polycarbonates; poly(pseudo amino acids); poly(amino acids); poly(hydroxyalkanoate)s; polyalkylene oxalates; polyoxaesters; polyanhydrides; polyortho esters; and copolymers, block copolymers, homopolymers, blends, and combinations thereof. In certain embodiments, the biocompatible substrate may be formed using a combination of bioabsorbable and non-bioabsorbable polymers.

Some non-limiting examples of suitable non-bioabsorbable materials include polyolefins, such as polyethylene and polypropylene including atactic, isotactic, syndiotactic, and blends thereof; polyethylene glycols; polyethylene oxides; ultra high molecular weight polyethylene; copolymers of polyethylene and polypropylene; polyisobutylene and ethylene-alpha olefin copolymers; fluorinated polyolefins, such as fluoroethylenes, including expanded polytetrafluoroethylene (ePTFE) and condensed polytetraflouroethylene c(PTFE), fluoropropylenes, fluoroPEGSs, and polytetrafluoroethylene; polyamides, such as nylon and polycaprolactam; polyamines; polyimines; polyesters, such as polyethylene terephthalate and polybutylene terephthalate; aliphatic polyesters; polyethers; polyether-esters, such as polybutester; polytetramethylene ether glycol; 1,4-butanediol; polyurethanes; acrylic polymers and copolymers; modacrylics; vinyl halide polymers and copolymers, such as polyvinyl chloride; polyvinyl alcohols; polyvinyl ethers, such as polyvinyl methyl ether; polyvinylidene halides, such as polyvinylidene fluoride and polyvinylidene chloride; polyacrylonitrile; polyaryletherketones; polyvinyl ketones; polyvinyl aromatics, such as polystyrene; polyvinyl esters, such as polyvinyl acetate; copolymers of vinyl monomers with each other and olefins, such as etheylene-methyl methacrylate copolymers, acrylonitrile-styrene copolymers, ABS resins, and ethylene-vinyl acetate copolymers; alkyd resins; polycarbonates; polyoxymethylenes; polyphosphazine; polyimides; epoxy resins; aramids, rayon; rayon-triacetate; spandex; silicones; and combinations thereof

The biocompatible substrates may be formed using any method within the purview of those skilled in the art. Some non-limiting examples include, weaving, knitting, braiding, crocheting, extruding, spraying, casting, molding, and combinations thereof. In some embodiments, the biocompatible substrate may be a two or three dimensional surgical mesh which is woven, knitted, braided, or crocheted from at least one first filament to form the substrate. In certain embodiments, the biocompatible substrate may be a surgical mesh consisting of at least one first filament made of polyethylene terephthalate.

The tissue-gripping elements, i.e., the barbed loops and/or the barbed and spiked naps, which are positioned on at least a portion of the biocompatible substrate, may be formed from at least one second filament. The second filaments may be made from any biocompatible, bioabsorbable, or non-bioabsorbable material, including those described herein. In some embodiments, the first and second filaments may be made from the same materials. In other embodiments, the first and second filaments may be made from different materials. For example, in some embodiments, the biocompatible substrate may be formed from at least one first filament made from a non-bioabsorbable material, i.e., polypropylene, and the tissue-gripping elements may be formed from at least one second filament made from a bioabsorbable material, i.e., polylactic acid.

The tissue-gripping elements, whether loops or spiked naps, include a plurality of barbs positioned along the length of the element. The barbs may be disposed in various arrangements along the length of the filament. The barbs may be formed using any suitable method, including but not limited to, injection molding, stamping, cutting, laser, ultrasonics, and the like. The barbs may be uni-directional, multi-directional, symmetrical, non-symmetrical, and combinations thereof.

The second filaments used to form the tissue-gripping elements may be barbed at any time during the manufacturing of the implants described herein. In some embodiments, the second filaments may be barbed prior to being incorporated into the biocompatible substrate. In some embodiments, the second filaments may be barbed after being incorporated into the biocompatible substrate. In still other embodiments the second filaments may be barbed while being incorporated into the biocompatible substrate.

In certain embodiments, the tissue-gripping elements may be made form second filaments added to the substrate as loops which extend from the surface of a biocompatible surface in a generally perpendicular manner. In other embodiments, the tissue-gripping elements may be made from a plurality of second filaments which individually extend from the surface of a biocompatible surface in a generally perpendicular manner. By generally perpendicular the tissue-gripping elements may protrude from the surface of the implant at about 90 degrees. It is envisioned that the tissue-gripping elements may protrude from the surface of the implant from about 75 to about 105 degrees.

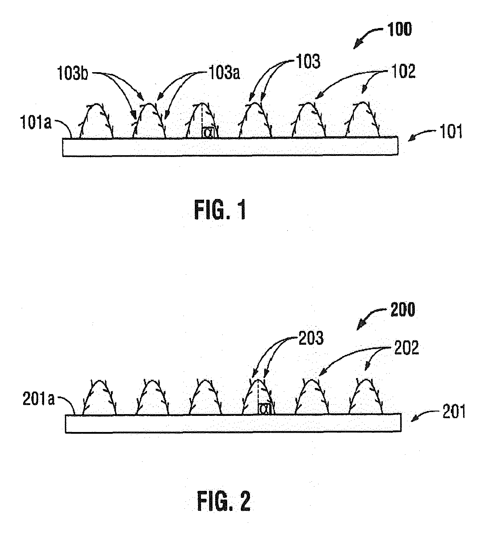

Referring now to FIG. 1 which illustrates implantable medical device 100 containing biocompatible substrate 101 having surface 101 a. At least one barbed loop 102 protrudes from surface 101 of the substrate 101 in a generally perpendicular orientation. As shown in Fig. 1, angle α is about 90° thus illustrating the generally perpendicular relationship between substrate 101 and barbed loops 102. Barbed loops 102 include a plurality of barbs 103 which may be all oriented in a single direction along the second filament which comprises the loops. Although barbs 103 are unidirectional, in the looped configuration, some of barbs 103 may be tissue-gripping barbs 103a and some of barbs 103 may not be tissue-gripping barbs 103b.

In FIG. 2, similar to FIG. 1, implantable medical device 200 includes biocompatible substrate 201 having surface 201a and at least one barbed loop 202. However, barbed loops 202 include a plurality of barbs 203 which may be oriented in more than one direction or multi-directional along the second filament which comprises the loops. All of barbs 203 may be tissue gripping. Angle α is about 90°, illustrating the generally perpendicular relationship between the substrate and the loops.

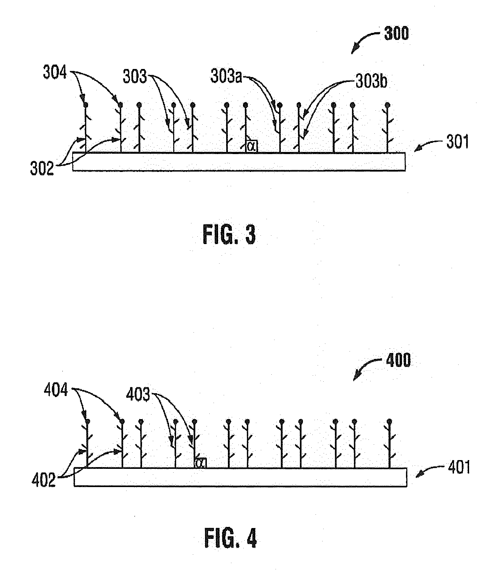

In FIGS. 1 and 2, the tissue-gripping element is shown as a barbed loop, 103 and 203, respectively. However, in some embodiments, such as those shown in FIGS. 3 and 4, the tissue-gripping elements may be barbed and spiked naps.

As depicted in FIG. 3, implantable medical device 300 includes biocompatible substrate 301 having surface 301a and at least one barbed and spiked nap 302 protruding from the surface of the substrate in a perpendicular manner. Naps 302 are substantially rectilinear in shape and include barbs 303 and spikes 304. Naps 302 may be formed from barbed loops in which the barbs were oriented in a single direction along the body of the loop. Barbs 303a may be tissue gripping while barbs 303b may not be tissue-gripping. Angle α is about 90°, illustrating the perpendicular relationship between the substrate and the naps. Spikes 304 are slightly greater in width than the remainder of the naps, providing additional tissue gripping capability to the barbed naps.

FIG. 4 illustrates, implantable medical device 400 having biocompatible substrate 401, having surface 401a and at least one barbed and spiked nap 402. Nap 402 includes barbs 403 and spikes 404. Naps 402 were formed from barbed loops in which the barbs were oriented in at least two distinct directions along the body of the filament which comprised the loops. Thus, all of barbs 403 may be tissue gripping.

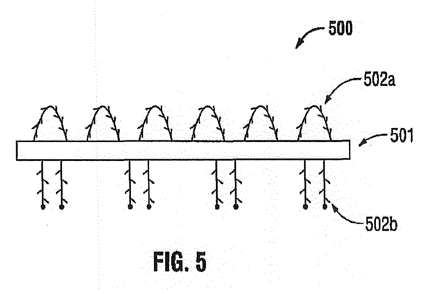

As shown in FIG. 5, 6, and 7, the implantable medical devices described herein may include any number, pattern or concentration of tissue-gripping elements. For example, in FIG. 5, implantable medical device 500 includes biocompatible substrate 501 having at least one barbed loop 502a and at least one barbed and spiked nap 502b. Although shown on opposite sides of substrate 501, it is envisioned that the combination of two different tissue-gripping elements may also be positioned on the same side and/or in any combination, concentration or pattern.

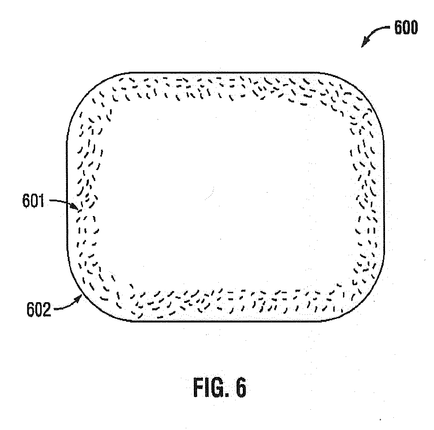

FIG. 6 illustrates a top view of an implantable medical device 600 that is planar in configuration, having a height, width and length. In this embodiment, gripping elements 601 are a contiguous part of biocompatible substrate 602 and are arranged along an outer perimeter of substrate 602. It is envisioned that in other embodiments the gripping elements may comprise the entire planar surface of the implant. In still other embodiments, the gripping elements may be arranged only at the corners of the implant. In yet another embodiment, the concentration of tissue-gripping elements may vary along different portions of the substrate. Other arrangements of the gripping element are possible and should be apparent to one skilled in the art.

Although the substrate is shown to be generally rectangular, the substrates described herein may be of any shape including elliptical, square, triangular, hexagonal, and circular and the like. In addition, the substrate may include apertures to accommodate the passage of bodily tissue when implanted. The implant can be shaped and sized during manufacturing or can be cut to a particular size and shape immediately before use.

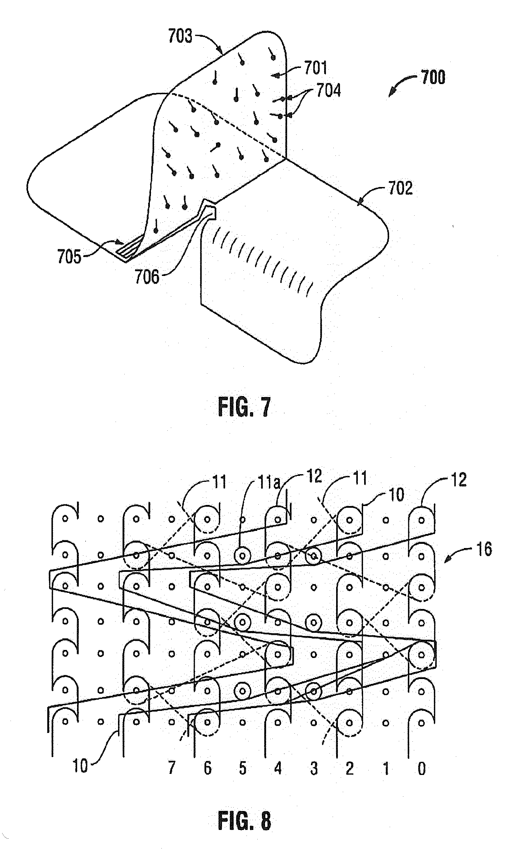

Turning to FIG. 7, which shows implantable medical device 700 including biocompatible substrate 702 including aperture 706 and flap 703 attached to substrate 702 via interface 705. Tissue-gripping elements 704 are shown positioned on flap 703 which is separate from substrate 702. Tissue-gripping elements 704 may be useful in securing flap 703 to portions of substrate 702. Flap 703 is attached to substrate 702 at interface 705 by stitching, welding, adhesive, and stapling or any other suitable method.

In certain embodiments, the implantable medical device may be a surgical mesh which made from a plurality of first and second filaments woven in any suitable manner that allows the filaments to form a substrate and form loops or naps which extend from the surface of said substrate. FIG. 8 diagrams one representative pattern that will form loops in accordance with the present disclosure. The implantable medical device may be made on a warp knitting machine, of` the tricot or Raschel type, with at least three sheets or warps of yarn and as many guide bars.

The front and intermediate guide-bars may be threaded with a first set of filaments or yarns. The intermediate bars may be threaded, one guide full, three guides empty, with monofilament or multifilament yarn. This yarn may be made from any suitable biocompatible material; and in some embodiments, may be made from polyethylene terephthalate. This filament or yarn is represented by a broken line and by reference number 11 in FIG. 8. The intermediate bar works in such a way as to obtain a zigzag openwork pattern between the columns of meshes.

The front bar is threaded; one guide full, one guide empty, and works in chain weave with a multifilament or monofilament yarn, represented by number 12 in FIG. 8. The chain stitch imprisons the monofilament 10 and maintains the knit in length while contributing to the formation of the knit with the intermediate sheet formed by yarn 11.

The rear bar may be threaded, one guide full and one guide empty, with a second filament, i.e., monofilament or multifilament. This second filament or yarn may be made from any suitable biocompatible material; and in some embodiments, may be made from polylactic acid. The second filament may be woven to form the barbed loops or the barbed and spiked naps of the final product.

The diameter of the second filament is over 0.10 millimeter. In practice, this diameter is between 0.14 and 0.18 millimeter and is of the order of 0.15 millimeter. This yarn or filament is represented by reference number 10 and in a solid line in FIG. 8.

The different filaments may be worked according to the following chart:

| Warp → | Rear bar I | Intermediate bar II | Front bar III |
|---|---|---|---|
| Rachel → | Front bar II | | Rear bar III |
| | | Intermediate bar II | |
| | 7 | 3 | 1 |
| | 7 | 2 | 0 |
| | 3 | 4 | 0 |
| | 4 | 5 | 1 |
| | 0 | 1 | |
| | 0 | 0 | |
| | 4 | 2 | |
| | 3 | 3 | |
| | | 1 | |
| | | 0 | |
| | | 4 | |
| | | 5 | |

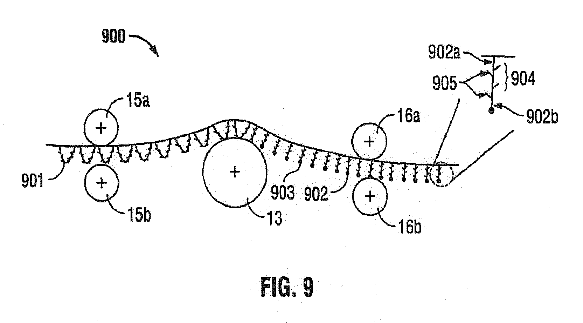

The rear bar places the yarn in partial weft under the chain stitch and "thrown" onto the needle not forming a chain stitch. For this reason, at the next row, the needle not forming a chain stitch not being supplied permits escape of the monofilament mesh which forms a loop (see FIG. 9) projecting from the front face of the medical device.

The medical device thus obtained may be a knit provided with loops which are generally perpendicular to one of the surfaces of the substrate. The loops also display the rigidity to hold at about a right angle, which is obtained by the rigidity or nerve of the second filament employed. This rigidity or nerve may be necessary for the subsequent formation of the spiked and barbed naps or barbed loops which ensure a tissue-gripping function.

Other patterns by which to obtain a knit with loops that protrude from one face should be apparent to one skilled in the art. In embodiments, the second filament used to form the loops can be cut along its length prior to the knitting of the substrate to form barbs. In other embodiments, the second filaments used to form the loops can first be knitted into the substrate and then may be cut along the length of the loops to form barbs.

FIG. 9 illustrates one method by which barbed loops 901 can be converted into spiked and barbed naps 902. In one embodiment, the method includes passing substrate 900 with loops 901 over cylinder 13 containing an electrical heating resistor. Substrate 900 may be pressed flat on cylinder 13 by two pairs of rollers, upstream 15a, 15b and downstream 16a, 16b, respectively, which may be vertically displaceable for controlling the pressing force. This control as well as that of the temperature of the resistor placed in cylinder 13 and of the speed of movement of substrate 900 across cylinder 13 make it possible to melt the head of each of the barbed loops 901 so that each barbed loop 901 forms two barbed and spiked naps 902.

Each spiked nap 902 thus has a substantially rectilinear body 904 protruding perpendicularly with respect to the substrate 900. Rectilinear body 904 includes attached end 902a and free end 902b, with free end 902b having spike 903 of greater width than that of the body 904 and barbs 905 positioned between attached end 902a and free end 902b. Spike 903 may have the shape of a sphere or mushroom.

In embodiments, the substrate and/or loops or naps of the medical device can be coated with a bioactive agent. The term "bioactive agent", as used herein, is used in its broadest sense and includes any substance or mixture of substances that have clinical use. Consequently, bioactive agents may or may not have pharmacological activity per se, e.g., a dye. Alternatively a bioactive agent could be any agent that provides a therapeutic or prophylactic effect, a compound that effects or participates in tissue growth, cell growth, cell differentiation, and an anti-adhesive compound, a compound that may be able to invoke a biological action such as an immune response, or could play any other role in one or more biological processes. It is envisioned that the bioactive agent may be applied to the substrate and/or loops or naps in any suitable form, e.g., films, powders, liquids, gels, and the like.

Examples of classes of bioactive agents, which may be utilized in accordance with the present disclosure include: anti-adhesives; antimicrobials; analgesics; antipyretics; anesthetics; antiepileptics; antihistamines; anti-inflammatories; cardiovascular drugs; diagnostic agents; sympathomimetics; cholinomimetics; antimuscarinics; antispasmodics; hormones; growth factors; muscle relaxants; adrenergic neuron blockers; antineoplastics; immunogenic agents; immunosuppressants; gastrointestinal drugs; diuretics; steroids; lipids; lipopolysaccharides; polysaccharides; platelet activating drugs; clotting factors; and enzymes. It is also intended that combinations of bioactive agents may be used.

Anti-adhesive agents can be used to prevent adhesions from forming between the mesh and the surrounding tissues opposite the target tissue. In addition, anti-adhesive agents may be used to prevent adhesions from forming between the coated implantable medical device and the packaging material. Some examples of these agents include, but are not limited to hydrophilic polymers such as poly(vinyl pyrrolidone), carboxymethyl cellulose, hyaluronic acid, polyethylene oxide, poly vinyl alcohols, and combinations thereof.

Suitable antimicrobial agents which may be included as a bioactive agent include: triclosan, also known as 2,4,4'-trichloro-2'-hydroxydiphenyl ether, chlorhexidine and its salts, including chlorhexidine acetate, chlorhexidine gluconate, chlorhexidine hydrochloride, and chlorhexidine sulfate, silver and its salts, including silver acetate, silver benzoate, silver carbonate, silver citrate, silver iodate, silver iodide, silver lactate, silver laurate, silver nitrate, silver oxide, silver palmitate, silver protein, and silver sulfadiazine; polymyxin, tetracycline; aminoglycosides, such as tobramycin and gentamicin; rifampicin; bacitracin; neomycin; chloramphenicol; miconazole; quinolones such as oxolinic acid, norfloxacin, nalidixic acid, pefloxacin, enoxacin and ciprofloxacin; penicillins such as oxacillin and pipracil, nonoxynol 9, fusidic acid, cephalosporins; and combinations thereof. In addition, antimicrobial proteins and peptides such as bovine lactoferrin and lactoferricin B may be included as a bioactive agent.

Other bioactive agents, which may be included as a bioactive agent include: local anesthetics; non-steroidal antifertility agents; parasympathomimetic agents; psychotherapeutic agents; tranquilizers; decongestants; sedative hypnotics; steroids; sulfonamides; sympathomimetic agents; vaccines; vitamins; antimalarials; anti-migraine agents; anti-parkinson agents such as L-dopa; anti-spasmodics; anticholinergic agents (e.g., oxybutynin); antitussives; bronchodilators; cardiovascular agents, such as coronary vasodilators and nitroglycerin; alkaloids; analgesics; narcotics such as codeine, dihydrocodeinone, meperidine, morphine and the like; non-narcotics, such as salicylates, aspirin, acetaminophen, d-propoxyphene and the like; opioid receptor antagonists, such as naltrexone and naloxone; anti-cancer agents; anticonvulsants; anti-emetics; antihistamines; anti-inflammatory agents, such as hormonal agents, hydrocortisone, prednisolone, prednisone, non-hormonal agents, allopurinol, indomethacin, phenylbutazone and the like; prostaglandins and cytotoxic drugs; chemotherapeutics, estrogens; antibacterials; antibiotics; anti-fungals; anti-virals; anticoagulants; anticonvulsants; antidepressants; antihistamines; and immunological agents.

Other examples of suitable bioactive agents, which may be included in the mesh or suture include: viruses and cells; peptides, polypeptides and proteins, as well as analogs, muteins, and active fragments thereof; immunoglobulins; antibodies; cytokines (e.g., lymphokines, monokines, chemokines); blood clotting factors; hemopoietic factors; interleukins (IL-2, IL-3, IL-4, IL-6); interferons (β-IFN, α-IFN and γ-IFN); erythropoietin; nucleases; tumor necrosis factor; colony stimulating factors (e.g., GCSF, GM-CSF, MCSF); insulin; anti-tumor agents and tumor suppressors; blood proteins such as fibrin, thrombin, fibrinogen, synthetic thrombin, synthetic fibrin, synthetic fibrinogen; gonadotropins (e.g., FSH, LH, CG, etc.); hormones and hormone analogs (e.g., growth hormone); vaccines (e.g., tumoral, bacterial and viral antigens); somatostatin; antigens; blood coagulation factors; growth factors (e.g., nerve growth factor, insulin-like growth factor); bone morphogenic proteins; TCF-B; protein inhibitors; protein antagonists; protein agonists; nucleic acids, such as antisense molecules, DNA, RNA, RNAi; oligonucleotides; polynucleotides; and ribozymes.

The barbed implantable medical devices described herein may be formed using any suitable method known to those skilled in the art. In certain embodiments, one such method may include: providing at least one barbed, biocompatible filament; and combining the at least one barbed biocompatible filament with a biocompatible substrate to form barbed loops along a surface of the biocompatible substrate. In other embodiments, a method may include: providing a biocompatible substrate having loops protruding perpendicularly from a surface of the biocompatible substrate; and forming barbs on the loops of the medical device.

In addition, the barbed loops may be treated in any manner suitable to separate the barbed loops into two separate barbed and spiked naps. For example, it may be useful to apply a certain amount of heat and/or pressure to melt the barbed loop thereby separating the loop into two separate naps and by melting the material used to form the loop, the ends of each separate nap will include a spike thus creating a spiked and barbed nap. The barbed loops may be treated using any suitable method, including heated rollers or cylinders, lasers, ovens, ultrasonics, and the like.

It will be apparent from the foregoing that, while particular forms of the implantable medical devices have been illustrated and described, various modifications can be made without departing from the spirit and scope of the present disclosure. For example, although particular barb configurations may be illustrated and described herein, any suitable configuration and arrangement may be possible.

The invention may be described by reference to the following numbered paragraphs:-
1. An implantable medical device comprising a biocompatible substrate having a surface comprising barbed loops.
2. The implantable medical device of paragraph 1 wherein the barbed loops are oriented perpendicularly to the surface of the biocompatible substrate.
3. The implantable medical device of paragraph 1 or paragraph 2 wherein the biocompatible substrate comprises a bioabsorbable material selected from the group consisting of polylactides, poly(lactic acid), polyglycolides, poly(glycolic acid), poly(trimethylene carbonate), poly(dioxanone), poly(hydroxybutyric acid), poly(hydroxyvaleric acid), poly(lactide-co-(e-caprolactone-)), poly(glycolide-co-(s-caprolactone)), polycarbonates, poly(pseudo amino acids), poly(amino acids), poly(hydroxyalkanoate)s, polyalkylene oxalates, polyoxaesters, polyanhydrides, polyortho esters, and copolymers, block copolymers, homopolymers, blends, and combinations thereof; or wherein the biocompatible substrate comprises a non-bioabsorbable material selected from the group consisting of at least one of polypropylene, polyethylene terephthalate, expanded polytetrafluoroethylene, condensed polytetrafluoroethylene and combinations thereof.
4. The implantable medical device of any preceding paragraph wherein the biocompatible substrate is a surgical mesh, patch, buttress, or pledget; preferably wherein the biocompatible substrate comprises a surgical mesh.
5. The implantable medical device of any preceding paragraph wherein the biocompatible substrate further comprises at least one flap; preferably wherein the at least one flap comprises barbed loops.
6. The implantable medical device of any preceding paragraph wherein the barbed loops are made of a bioabsorbable polymer; preferably wherein the bioabsorbable polymer is selected from the group consisting of p-dioxanone, polyglycolides, polyorthoesters, polymer of trimethylene carbonate, sterocopolymers of L-lactic acid, copolymers of lactic acid and a compatible comonomer, such as derivatives of alpha-hydroxy acids and combinations thereof; preferably still wherein the bioabsorbable polymer comprises polylactic acid.
7. The implantable medical device of any of paragraphs 1 to 5 wherein the barbed loops are made of a non-bioabsorbable polymer; preferably wherein the non-bioabsorbable polymer is selected from the group consisting of polypropylene, polyethylene tercphthalate, expanded polytetrafluoroethylene, condensed polyletrafluoroethylene and combinations thereof.
8.The implantable medical device according to paragraph 6 or paragraph 7, wherein the barbed loops comprise uni-directional barbs, and/or wherein the barbed loops comprise multi-directional barbs.
9. The implantable medical device of any preceding paragraph further comprising at least one bioactive agent; preferably wherein the bioactive agent comprises an anesthetic.
10. An implantable medical device comprising a biocompatible substrate having a surface comprising barbed and spiked naps.
11. The implantable medical device of paragraph 10, wherein the barbed and spiked naps are oriented perpendicularly to the surface of the biocompatible substrate; and/or wherein the barbed and spiked naps comprise a substantially rectilinear body, a free end having a head of greater width than that of said rectilinear body, and barbs that protrude from said rectilinear body in the portion between the free end and an end attached to the substrate.
12. The implantable medical device of paragraph 10 or paragraph 11 wherein the biocompatible substrate comprises a bioabsorbable material selected from the group consisting of polylactides, poly(lactic acid), polyglycolides, poly(glycolic acid), poly(trimethylene carbonate), poly(dioxanone), poly(hydroxybutyric acid), poly(hydroxyvaleric acid), poly(lactide-co-(e-caprolactone-)), poly(glycolide-co-(e-caprolactone)), polycarbonates, poly(pseudo amino acids), poly(amino acids), poly(hydroxyalkanoate)s, polyalkylene oxalates, polyoxaesters, polyanhydrides, polyortho esters, and copolymers, block copolymers, homopolymers, blends, and combinations thereof; or wherein the biocompatible substrate comprises a non-bioabsorbable material selected from the group consisting of at least one of polypropylene, polyethylene terephthalate, expanded polytetrafluoroethylene, condensed polytetrafluoroethylene and combinations thereof.
13. The implantable medical device of any of paragraphs 10 to 12 wherein the biocompatible substrate is a surgical mesh, patch, buttress, or pledget; preferably wherein the biocompatible substrate comprises a surgical mesh.
14. The implantable medical device of any of paragraphs 10 to 13 wherein the biocompatible substrate further comprises at least one flap; preferably wherein the at least one flap comprises barbed and spiked naps; preferably still wherein the barbed and spiked naps are made of a bioabsorbable polymer wherein the bioabsorbable polymer is selected from the group consisting of p-dioxanonc, polyglycolides, polyorthoesters, polymer of trimethylene carbonate, sterocopolymers of L-lactic acid, copolymers of lactic acid and a compatible comonomer, such as derivatives of alpha-hydroxy acids and combinations thereof; still preferably wherein the bioabsorbable polymer comprises polylactic acid; or the implantable medical device of any of paragraphs 10 to 13 wherein the barbed and spiked naps are made of a non-bioabsorbable polymer; preferably wherein the non-bioabsorbable polymer is selected from the group consisting of polypropylene, polyethylene terephthalate, expanded polytetrafluoroethylene, condensed polytetrafluoroethylene and combinations thereof.
15. The implantable medical device of any of paragraphs 10 to 14 wherein the barbed and spiked naps comprise uni-directional barbs and/or wherein the barbed and spiked naps comprise multi-directional barbs.
16. The implantable medical device of any of paragraphs 10 to 15 further comprising at least one bioactive agent; preferably wherein the bioactive agent comprises an anesthetic.
17. A method of forming a barbed implantable medical device comprising:
   providing at least one barbed, biocompatible filament; and
   combining the at least one barbed biocompatible filament with a biocompatible substrate to form barbed loops along a surface of the biocompatible substrate.

## Claims

1. An implantable medical device comprising a biocompatible substrate having a surface comprising at least one barbed and spiked nap, wherein the nap has a free end, **characterised in that**:
the spike is formed on the free end of the nap.

2. The implantable medical device of claim 1 wherein the barbed and spiked naps are oriented perpendicularly to the surface of the biocompatible substrate.

3. The implantable medical device of claim 1 or claim 2 wherein the barbed and spiked naps comprise a substantially rectilinear body, a free end having a head of greater width than that of said rectilinear body, and barbs that protrude from said rectilinear body in the portion between the free end and an end attached to the substrate.

4. The implantable medical device of any preceding claim wherein the biocompatible substrate comprises a bioabsorbable material selected from the group consisting of polylactides, poly(lactic acid), polyglycolides, poly(glycolic acid), poly(trimethylene carbonate), poly(dioxanone), poly(hydroxybutyric acid), poly(hydroxyvaleric acid), poly(lactide-co-(e-caprolactone-)), poly(glycolide-co-(s-caprolactone)), polycarbonates, poly(pseudo amino acids), poly(amino acids), poly(hydroxyalkanoate)s, polyalkylene oxalates, polyoxaesters, polyanhydrides, polyortho esters, and copolymers, block copolymers, homopolymers, blends, and combinations thereof

5. The implantable medical device according to any of claims 1 to 3, wherein the biocompatible substrate comprises a non-bioabsorbable material selected from the group consisting of at least one of polypropylene, polyethylene terephthalate, expanded polytetrafluoroethylene, condensed polytetrafluoroethylene and combinations thereof.

6. The implantable medical device of any preceding claim wherein the biocompatible substrate is a surgical mesh, patch, buttress, or pledget; preferably wherein the biocompatible substrate comprises a surgical mesh.

7. The implantable medical device of any preceding claim wherein the biocompatible substrate further comprises at least one flap.

8. The implantable medical device of any preceding claim wherein the barbed and spiked naps are made of a bioabsorbable polymer; preferably wherein the bioabsorbable polymer is selected from the group consisting of p-dioxanone, polyglycolides, polyorthoesters, polymer of trimethylene carbonate, sterocopolymers of L-lactic acid, copolymers of lactic acid and a compatible comonomer, such as derivatives of alpha-hydroxy acids and combinations thereof; preferably still wherein the bioabsorbable polymer comprises polylactic acid.

9. The implantable medical device of any of claims 1 to 7 wherein the barbed and spiked naps are made of a non-bioabsorbable polymer; preferably wherein the non-bioabsorbable polymer is selected from the group consisting of polypropylene, polyethylene tercphthalate, expanded polytetrafluoroethylene, condensed polyletrafluoroethylene and combinations thereof.

10. The implantable medical device of any preceding claim comprising a plurality of first and second filaments, wherein the second filament is barbed.

11. The implantable medical device of claim 10 wherein first and second filaments are made from the same materials.

12. The implantable medical device of claim 10 wherein first and second filaments are made from different materials; preferably wherein the first filament is made from a non-bioabsorbable material and the second filament is made from a bioabsorbable material; preferably wherein the bioabsorbable material is polylactic acid and the non-bioabsorbable material is polypropylene.

13. The implantable medical device according to any of claims 10 to 12 wherein the diameter of the second filament is of the range 0.14 mm to 0.18 mm; preferably wherein the diameter of the second filament is of the order of 0.15 mm.

14. The implantable medical device according to any preceding claim, wherein the barbed and spiked naps comprise uni-directional barbs, and/or wherein the barbed and spiked naps comprise multi-directional barbs.

15. The implantable medical device of any preceding claim further comprising at least one bioactive agent; preferably wherein the bioactive agent is an anaesthetic and/or an anti-adhesive.

16. The implantable medical device of any preceding claim further comprising at least one barbed loop.

17. A method of forming a barbed implantable medical device comprising:
providing a first, barbed biocompatible filament;
combining the at least one barbed biocompatible filament with a biocompatible substrate to form barbed loops along a surface of the biocompatible substrate; and
separating the barbed loops into barbed and spiked naps, **characterised in that**:
the spikes are formed at the free end of the barbed and spiked naps.
